# FASCICULE DE BREVET EUROPEEN

(11) **EP 2 200 680 B1**
(45) Date de publication et mention de la délivrance du brevet: **04.06.2014**
(21) Numéro de dépôt: 08845427.7
(22) Date de dépôt: 22.10.2008
(51) Int. Cl.: A61M 5/32

(54) **DISPOSITIF DE SECURITE POUR UNE SERINGUE**
SICHERHEITSGERÄT FÜR EINE SPRITZE
SAFETY DEVICE FOR A SYRINGE

(30) Priorité: 23.10.2007 FR 0758495
(43) Date de publication de la demande: 30.06.2010
(73) Titulaire: Tech Group Europe Limited, Dublin 25 (IE)
(72) Inventeur: CHEVALLIER, Stéphane, F-77165 Saint-Soupplets (FR); CHEVALLIER, Jean-Michel, F-95880 Enghien-Les-Bains (FR)
(74) Mandataire: Lippert, Stachow & Partner
(86) Numéro de dépôt international: PCT/FR2008/051907
(87) Numéro de publication internationale: WO 2009/056734

(56) Documents cités:
- WO-A-98/35714
- WO-A-2005/039678
- US-A1- 2003 229 314
- US-A1- 2005 080 383
- US-B1- 6 585 702

## Description

La présente invention concerne un dispositif de sécurité pour une seringue ayant un corps de seringue, un piston et une aiguille, le dispositif comprenant un manchon de support apte à retenir le corps de seringue par rapport à lui, un manchon de protection, et au moins une patte de retenue solidaire du manchon de protection et apte à occuper une position de retenue dans laquelle cette patte est retenue par rapport au manchon de support, et une position libérée dans laquelle ladite patte cesse d'être retenue par rapport au manchon de support pour permettre un coulissement relatif du manchon de protection et du manchon de support vers une position de protection dans laquelle l'aiguille est entourée par le manchon de protection, le piston présentant une jupe cylindrique apte, en fin de course d'injection dudit piston, à parvenir en regard d'une portion d'extrémité proximale du manchon de support pour coopérer avec une partie d'actionnement de la patte de retenue pour solliciter ladite patte vers sa position libérée.

Au sens de l'invention, l'extrémité proximale d'un élément est celle qui est la plus proche des doigts d'un utilisateur pratiquant une injection à l'aide du dispositif, tandis que l'extrémité distale est l'extrémité opposée.

Un dispositif de ce type est connu par les documents EP-A 235 603, EP-A 1 474 194 et WO 2005/039678.

Dans EP 1 235 603, la jupe cylindrique du piston est épaisse, de sorte qu'elle est rigide, et présente une forme intérieure tronconique permettant sa venue en contact avec les faces externes inclinées de deux pattes de retenue, pour ramener ces pattes vers l'intérieur, c'est-à-dire vers l'axe du dispositif, de manière à les libérer de leur retenue par rapport au manchon de support. Pour que la libération des pattes intervienne de manière fiable et systématique, il est nécessaire que la jupe soit épaisse et rigide et que les pattes soient accessibles pour cette jupe. Ainsi, les pattes sont relativement distantes de l'axe central du dispositif car elles sont situées de part et d'autre de la collerette du corps de seringue qui sert à retenir ce corps par rapport au manchon de support.

Il est donc nécessaire, avec un dispositif du type décrit dans EP 1 235 603, d'utiliser un piston ayant une jupe de conformation particulière et des dimensions diamétrales importantes.

Dans EP 1 474 194, la jupe présente la forme d'une portion de cylindre dont l'extrémité inférieure est légèrement chanfreinée du côté intérieur. Cette fois, les pattes de retenue du manchon de protection par rapport au manchon de support sont sensiblement contenues dans l'encombrement de la collerette du corps de seringue lorsqu'elles occupent leur position de retenue. En conséquence, ces pattes ne sont pas accessibles aisément pour la jupe du piston, de sorte que des pattes de relais, solidaires du manchon de support, sont nécessaires. En fin d'injection, la jupe vient ramener vers l'intérieur les pattes de relais, et ces pattes coopèrent avec les pattes de retenue du manchon de protection pour ramener ces dernières également vers l'intérieur.

Au total, l'encombrement du dispositif dans la direction diamétrale est relativement important.

La présente invention a pour but d'améliorer encore l'état de la technique précitée, en permettant de fabriquer la jupe du piston à moindre coût et d'assurer la libération de la ou des pattes de retenue par cette jupe en fin d'injection de manière sécurisée, tout en permettant que les dimensions diamétrales du dispositif soient aussi faibles que possible.

Ce but est atteint grâce au fait que la portion d'extrémité proximale du manchon de support présente au moins une rampe de déflexion apte, en fin de course du piston, à coopérer avec ladite jupe pour déformer cette dernière de manière à rapprocher une portion de ladite jupe de la partie d'actionnement de la patte de retenue pour favoriser la coopération entre la jupe et la partie d'actionnement.

La jupe peut avoir la forme d'une portion de paroi cylindrique, légèrement déformable radialement par pression. En fin de course d'injection, la rampe de déflexion déforme cette jupe pour assurer un contact fiable entre cette dernière et la partie d'actionnement de la patte de retenue. Contrairement à ce que représente EP 1 474 194, la jupe contacte ainsi directement la ou les pattes de retenue. Toutefois, ce contact est obtenu de manière fiable grâce à la présence de la rampe de déflexion. Ainsi, même si la ou les pattes de retenue sont au moins en partie contenues dans l'encombrement radial d'une collerette que présenterait le corps de seringue, la ou les pattes peuvent être ramenées dans leur position libérée par la jupe.

Contrairement à EP 1 235603, la jupe peut avoir des dimensions diamétrales réduites de manière à permettre l'actionnement du dispositif, c'est-à-dire le déplacement relatif du manchon de protection et du manchon de support, sans que la ou les pattes de retenue ne s'étendent à une distance importante de l'axe du dispositif, ni en particulier autour d'une collerette que présenterait le corps de seringue.

Avantageusement, au moins dans la position de retenue de la patte de retenue, la partie d'actionnement de cette patte de retenue est disposée sensiblement en regard de la rampe de déflexion.

Cette position permet d'assurer que la déformation de la jupe du piston nécessaire à la libération de la patte de retenue soit réalisée au bon endroit.

Avantageusement, la partie d'extrémité proximale du manchon de support forme une cavité cylindrique dans laquelle la jupe cylindrique du piston est apte à pénétrer en fin de course d'injection de ce dernier, et la rampe est formée sur au moins une portion de nervure annulaire disposée sur la paroi axiale interne de ladite cavité.

Ceci constitue une manière simple de réaliser la rampe de déflexion.

Avantageusement, la partie d'actionnement de la patte de retenue est formée sur un côté externe de la patte de retenue, et la rampe de déflexion est formée sur la périphérie interne du manchon de support.

Avantageusement, le corps de seringue présente une collerette située à l'extrémité proximale de ce corps et, au moins dans sa position de retenue, la patte de retenue s'étend entre cette collerette et l'extrémité distale du corps de seringue, et cette patte de retenue est au moins en partie située dans l'encombrement diamétral de la collerette lorsque cette patte est sollicitée par la jupe cylindrique vers sa position libérée.

En effet, malgré le fait que cette patte est au moins partiellement "masquée" par l'encombrement de la collerette, elle est accessible pour la jupe grâce à la déformation de cette dernière réalisée par la rampe de déflexion. Ceci permet de diminuer considérablement les dimensions diamétrales du dispositif.

Selon une disposition avantageuse, le manchon de protection est emmanché dans le manchon de support, et la patte de retenue est solidaire du manchon de protection et présente, sur sa périphérie externe, un décrochement de retenue permettant sa retenue par rapport à une nervure de retenue que présente le manchon de support.

Selon une autre disposition avantageuse, le manchon de support est emmanché dans le manchon de protection, la partie d'extrémité proximale du manchon de support présente une portion de paroi axiale externe sur laquelle est formée la rampe, et la partie d'actionnement de la patte de retenue est formée sur un côté externe de la patte de retenue.

L'invention sera bien comprise et ses avantages apparaîtront mieux à la lecture de la description détaillée qui suit, de modes de réalisation représentés à titre d'exemples non limitatifs. La description se réfère aux dessins annexés, sur lesquels :
- la figure 1 est une vue en perspective coupée dans un plan diamétral axial d'un dispositif conforme à l'invention, selon un premier mode de réalisation ;
- la figure 2 est une vue en coupe axiale de ce dispositif, prise perpendiculairement à la section de la figure 1, alors que le piston parvient vers la fin de l'injection ;
- la figure 3 est une vue agrandie de la zone Z de la figure 2, prise selon une coupe dans le plan III-III correspondant à celui de la section de la figure 1 ;
- la figure 4 est une vue dans la même coupe que la figure 3, montrant le dispositif en fin d'injection, alors que l'aiguille est protégée par le manchon de protection ; et
- la figure 5 est une vue partielle en coupe axiale d'un dispositif conforme à l'invention, selon un autre mode de réalisation, montrant deux positions de ce dispositif.

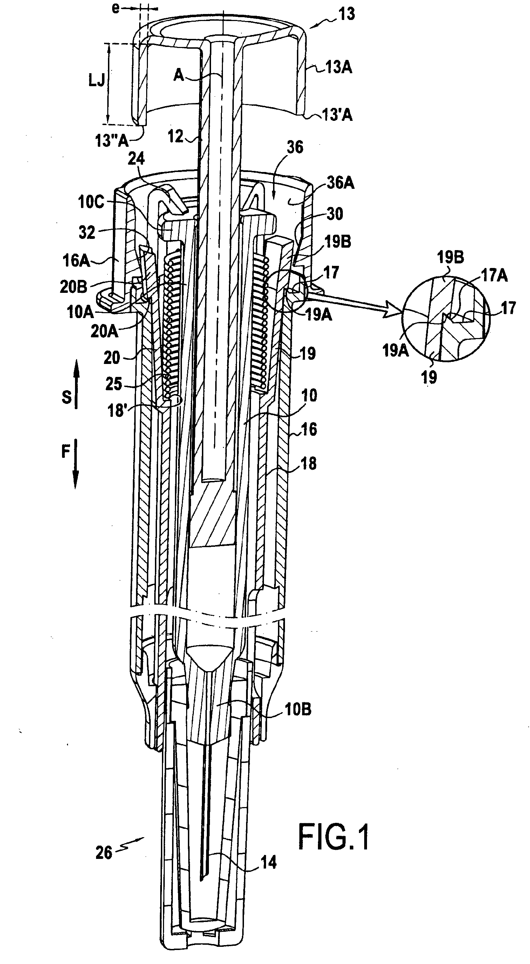

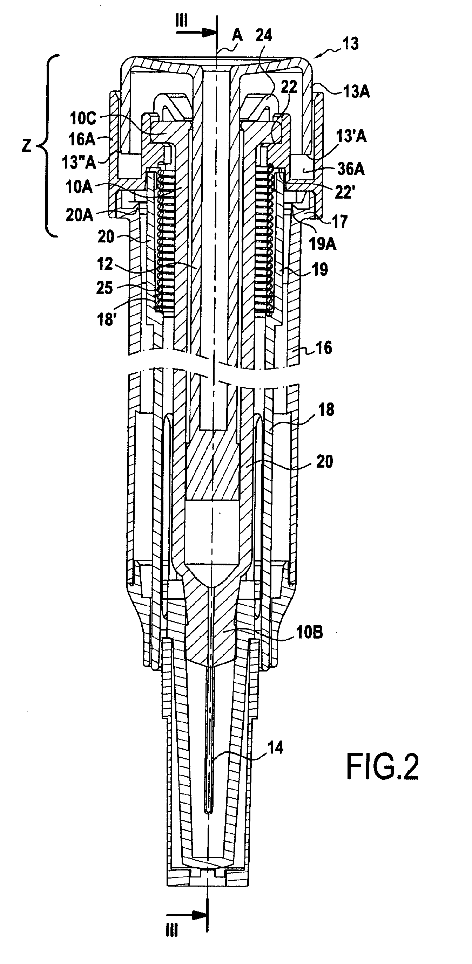

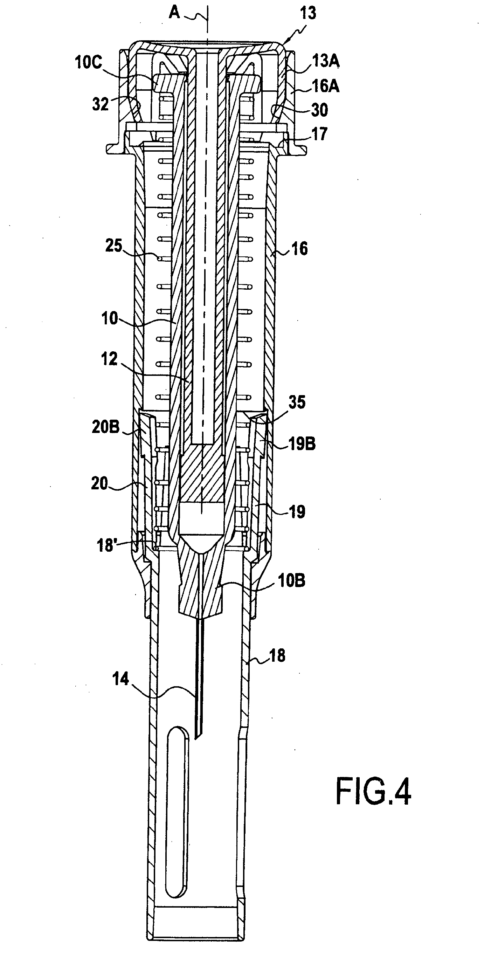

Le dispositif représenté sur les figures 1 à 4 comprend une seringue ayant un corps de seringue 10, un piston 12 qui peut coulisser dans ce corps, à partir d'une extrémité proximale 10A de ce dernier, entre une position d'attente dans laquelle il est représenté sur la figure 1 et une position de fin d'injection dans laquelle il est représenté sur la figure 4. A l'opposé du piston, c'est-à-dire à l'extrémité distale 10B du corps de seringue, une aiguille 14 est raccordée au corps. Le corps de seringue est préférentiellement en verre ou en plastique.

Le dispositif de support de sécurité pour cette seringue comprend, selon le premier mode de réalisation, un manchon de support 16 et un manchon de protection 18 qui est emmanché dans le manchon de support 16 et est agencé pour pouvoir coulisser par rapport à ce support. Dans la position d'attente avant l'injection, le manchon de protection 18 est retenu à l'intérieur du manchon de support 16 par deux pattes de retenue, respectivement 19 et 20, qui sont solidaires du manchon de protection 18 et sont retenues par rapport au manchon de support 16. En effet, ces pattes sont agencées pour pouvoir s'accrocher sur un épaulement 17 que présente le manchon de support au voisinage de son extrémité proximale 16A. Les pattes présentent à cet effet un décrochement, respectivement 19A et 20A, qui s'accroche sur le rebord de l'épaulement 17. Comme on le voit mieux sur la figure 1, ce rebord 17A est légèrement en saillie dans le sens S allant de l'extrémité distale vers l'extrémité proximale de manière à former une nervure de retenue. Les décrochements des pattes et ce rebord présentent la forme de rampes inclinées vers le haut pour favoriser l'accrochage et le décrochage des pattes de retenue.

Le corps de seringue est quant à lui retenu par rapport au manchon de support par la collerette 10C de ce corps, qui repose d'une part sur un épaulement 22 du manchon de support de manière à empêcher le déplacement du corps de seringue par rapport à ce manchon dans le sens d'injection F opposé au sens S, la collerette étant par ailleurs retenue vis-à-vis d'un déplacement dans le sens S par des pattes élastiques de maintien 24 qui sont solidaires du manchon de support 16. Un ressort de rappel 25 est disposé entre une première surface d'appui fixe par rapport au manchon de support, formée par la face inférieure 22' de l'épaulement 22 et une deuxième surface d'appui fixe par rapport au manchon de protection formée par un épaulement intérieur 18' de ce manchon. A son extrémité distale, le corps de seringue porte un capuchon 26 qui coiffe l'aiguille 14 et peut être ôté pour dégager cette aiguille de manière à permettre une injection.

Une fois l'ensemble constitué par le manchon de support 16, le manchon de protection 18 et le ressort 25 assemblé, le corps de seringue, portant le capuchon 26, peut être disposé à l'intérieur du manchon de protection par l'extrémité proximale de ce dernier, jusqu'à ce que la collerette vienne en butée sur l'épaulement 22 et soit retenue par les pattes 24 qui s'effacent par flexion élastique lors du passage de cette collerette.

Pour plus de détails, on pourra se reporter au document EP 1 474 194 qui montre un dispositif dont la configuration est sensiblement analogue à celle du dispositif de l'invention, pour ce qui vient d'être décrit.

On voit sur les figures 1, 3 et 4 que le manchon de support 16 présente, dans sa portion d'extrémité proximale, deux rampes de déflexion, respectivement 30 et 32 qui, lorsque le manchon de protection 18 est dans sa position escamotée à l'intérieur du manchon de support 16, c'est-à-dire lorsque les pattes de retenue 19 et 20 sont dans leur position de retenue, est disposé en regard des parties d'actionnement de ces pattes constituées par leurs têtes voisines de leurs extrémités libres, respectivement 19B et 20B.

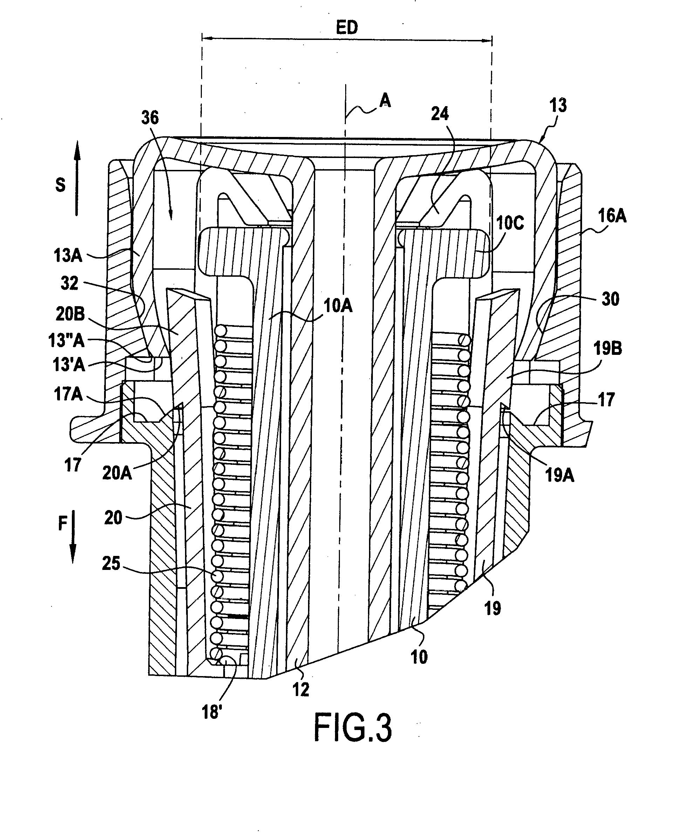

La tête 13 du piston présente une jupe cylindrique 13A qui, comme on le voit sur la figure 3, vient au contact des portions d'actionnement 19B et 20B des pattes de retenue 19 et 20 pour ramener ces dernières vers l'axe longitudinal central A du dispositif et faire ainsi échapper leurs décrochements 19A et 20A à l'épaulement 17. En d'autres termes, la coopération entre la jupe et les portions d'actionnement des pattes décroche ces pattes et permet ainsi l'avancement automatique du manchon de protection dans le sens F, vers l'extrémité distale, pour que le manchon vienne entourer l'aiguille 14 et protéger ainsi cette dernière.

Les rampes 30 et 32, et les portions d'actionnement 19B et 20B des pattes 19 et 20 sont en recouvrement axial. En conséquence, lorsque l'extrémité inférieure 13'A (c'est-à-dire l'extrémité distale) de la jupe 13A vient au contact des rampes 30 et 32, cette jupe se déforme localement de manière à se rapprocher de l'axe A, c'est-à-dire pour rapprocher la portion déformée en contact avec les rampes, de la partie d'actionnement des pattes de retenue, et favoriser ainsi la coopération entre la jupe et ces parties d'actionnement. En d'autres termes, les bords libres de la jupe en contact avec les rampes sont incurvés vers l'axe A pour favoriser le décrochage des pattes 19 et 20.

Il est avantageux que, comme dans l'exemple représenté, la ou les rampes d'inflexion forment une ou plusieurs portions discontinues d'une nervure annulaire. En effet, les discontinuités de cette nervure forment des espaces favorisant la déformation de la jupe. Ainsi, si deux rampes diamétralement opposées sont prévues, elles ont tendance à ovaliser le bord 13'A de la jupe qui, dans la région des rampes, définira des dimensions diamétrales inférieures à celles qu'aura la jupe dans les discontinuités de la nervure précitée. Ainsi, la jupe oppose une faible résistance à la déformation, et la poussée du piston dans le sens de l'injection reste aisée.

Le piston peut être réalisé en une seule pièce dans un matériau plastique du type couramment utilisé pour réaliser des pistons de seringue. Il s'agit par exemple de polyoléfine ou analogue. L'épaisseur de la jupe est choisie pour que celle-ci présente une intégrité mécanique tout en étant aisément déformable. En particulier, on pourra choisir que cette épaisseur e soit de l'ordre de 0,5 à 1,5 mm, de préférence de l'ordre de 1 mm, tandis que la longueur LJ de la jupe est de l'ordre de 1 cm.

On voit sur les figures que la partie d'extrémité proximale 16A du manchon de support 16 forme une cavité cylindrique 36 dans laquelle la jupe cylindrique 13A est apte à pénétrer en fin de course d'injection du piston. La ou les rampes d'inflexion sont formées sur une portion de nervure annulaire disposée sur la paroi axiale interne 36A de cette cavité. Par ailleurs, pour la ou les pattes de retenue, la partie d'actionnement 19B, 20B est formée sur un côté externe de la patte (c'est-à-dire sur un côté éloigné de l'axe A) tandis que la ou les rampes de déflexion 30, 32 sont formées sur la périphérie interne de l'élément qui les porte, en l'espèce le manchon de support 16.

La figure 3 montre la situation alors que l'extrémité 13'A de la jupe 13A coopère avec les parties d'actionnement 19B, 20B, des pattes de retenue 19, 20, juste avant que ces pattes ne se décrochent de l'épaulement 17. On voit alors que ces pattes sont en partie contenues dans l'encombrement diamétral ED de la collerette 10C. En d'autres termes, alors qu'elles sont sollicitées vers leur position libérée, les pattes viennent en partie se loger sous la collerette 10C, c'est-à-dire entre cette collerette et l'extrémité distale du corps de seringue. Malgré cela, les pattes restent accessibles pour être sollicitées par la jupe 13A grâce à la présence des rampes 30, 32. On peut ainsi minimiser l'encombrement diamétral global du dispositif.

On relève que l'extrémité libre 13'A de la jupe 13A présente un chanfrein 13"A de son côté externe. Ce chanfrein favorise le coulissement de cette extrémité libre sur les rampes 30 et 32, réduisant tout blocage lors de la poussée du piston.

La figure 4 montre le dispositif une fois que les pattes de retenue 19 et 20 ont été décrochées de l'épaulement 17, permettant ainsi la sortie du manchon de protection 18 sous l'effet de l'effort de rappel exercé par le ressort 25. On voit alors que la rentrée du fourreau de protection 18 à l'intérieur du fourreau de support 16 est empêchée par la coopération entre les extrémités libres des pattes 19 et 20 et un épaulement 35 de la paroi interne du manchon de support, tourné vers l'extrémité distale.

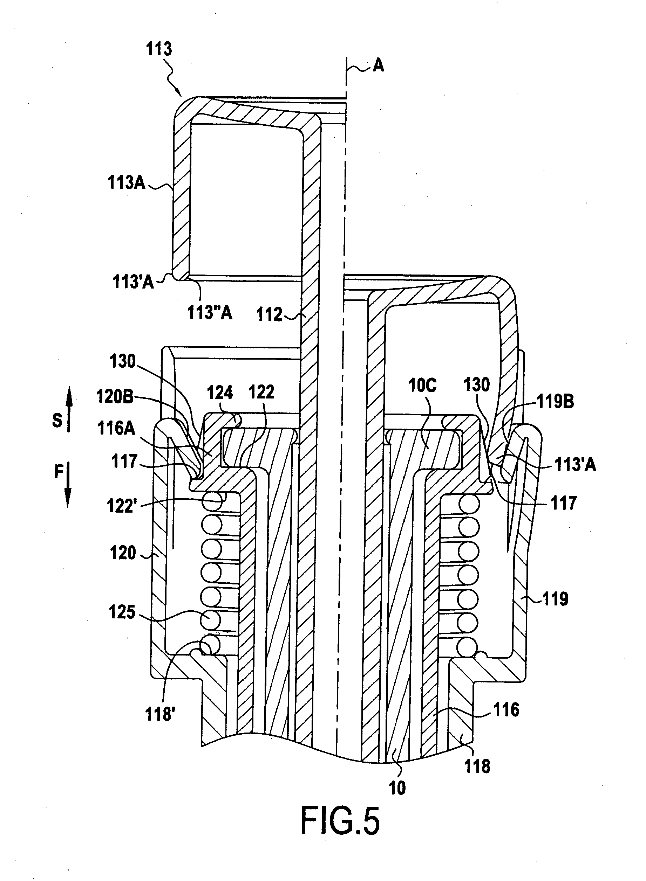

On décrit maintenant le mode de réalisation de la figure 5. Sur la moitié gauche de cette figure, le dispositif est représenté en cours d'injection, alors que les pattes de retenue sont dans leur position de retenue, tandis que, sur la moitié droite, les pattes de retenue sont en train d'être dégagées.

Dans ce mode de réalisation, le manchon de support 116 est disposé à l'intérieur du manchon de protection 118. Le corps de seringue 10 est retenu par rapport au manchon de support grâce au fait que la collerette 10C de ce corps est retenue entre un épaulement 122 tourné vers l'extrémité proximale et une nervure d'encliquetage ou analogue 124 espacée de cet épaulement. Cet épaulement et cette nervure sont solidaires du manchon 116 et, plus précisément, sont situés à son extrémité proximale 116A. Un ressort de rappel 125 est disposé entre la surface d'appui 122' qui est fixe par rapport au manchon 116 et est constitué par la face opposée de l'épaulement 122, et la surface d'appui 118' qui est fixe par rapport au manchon de protection 118 et est tournée vers l'extrémité proximale. Des pattes de retenue 119, 120 qui sont solidaires du manchon du protection 118 servent à retenir le manchon de protection par rapport au manchon de support.

Plus précisément, comme on le voit sur la moitié gauche de la figure, la patte de retenue 120 est en appui sur un épaulement 117 du manchon de support 116 qui est tourné vers l'extrémité proximale. En l'espèce, cette coopération se fait par l'extrémité libre de la patte qui est conformée en crochet et est dirigée vers l'extrémité distale. Ainsi, la patte s'accroche sur l'épaulement 117 et empêche le déplacement du fourreau de support dans le sens S opposé au sens d'injection F. Comme on le voit mieux sur la partie droite de la figure, l'épaulement 117 est adjacent à une rampe de déflexion 130 qui est disposée sur la périphérie axiale externe du manchon de support 116. En l'espèce, deux portions de rampe analogues sont disposées de part et d'autre de l'épaulement 117 qui est donc formé par la surface radiale d'un décrochement formé entre ces deux portions de rampe.

On voit sur la partie droite de la figure que, en fin d'injection, la jupe 113A du piston 112 coopère avec une partie d'actionnement 119B de la patte 119 pour fléchir cette dernière vers l'extérieur, dans le sens opposé à l'axe A, de manière à décrocher la patte de l'épaulement 117. Il en va évidemment de même pour la partie d'actionnement 120B de la patte 120. Ainsi, le manchon de support 116 et le corps de seringue 10 qu'il porte peuvent être déplacés par rapport au manchon de protection 118 dans le sens S opposé au sens d'injection F, jusqu'à ce que l'aiguille soit rentrée à l'intérieur de l'extrémité distale (non représentée) du manchon de protection 118.

Avantageusement, les pattes 119 et 120 sont formées dans des découpes de la paroi cylindrique du manchon de protection 118. Dans ce mode de réalisation également, les rampes 130 favorisent la déformation de la jupe 113A pour sécuriser sa coopération avec les parties d'actionnement 119B et 120B des pattes 119 et 120 et décrocher ainsi ces dernières de manière fiable. L'extrémité libre 113'A de la jupe 113A présente, sur sa face interne, un chanfrein 113"A favorisant le glissement de cette extrémité libre sur les rampes. Cette fois encore, la jupe 113A est ovalisée par son contact avec les rampes, mais ses dimensions diamétrales augmentent localement dans les zones de contact avec les rampes, tandis qu'elles diminuent dans une section transversale à celle qui est représentée.

## Revendications

1. Dispositif de sécurité pour une seringue ayant un corps de seringue (10), un piston (12 ; 112) et une aiguille (14), le dispositif comprenant un manchon de support (16 ; 116) apte à retenir le corps de seringue (10) par rapport à lui, un manchon de protection (18 ; 118), et au moins une patte de retenue (19, 20 ; 119, 120) solidaire du manchon de protection et apte à occuper une position de retenue dans laquelle cette patte est retenue par rapport au manchon de support, et une position libérée dans laquelle ladite patte cesse d'être retenue par rapport au manchon de support pour permettre un coulissement relatif du manchon de protection et du manchon de support vers une position de protection dans laquelle l'aiguille (14) est entourée par le manchon de protection (18 ; 118), le piston présentant une jupe cylindrique (13A ; 113A) apte, en fin de course d'injection dudit piston, à parvenir en regard d'une portion d'extrémité proximale (16A ; 116A) du manchon de support (16 ; 116) pour coopérer avec une partie d'actionnement (19B , 20B ; 119B, 120B) de la patte de retenue pour solliciter ladite patte vers sa position libérée,
**caractérisé en ce que** la portion d'extrémité proximale (16A ; 116A) du manchon de support (16 ; 116) présente au moins une rampe de déflexion (30, 32 ; 130) apte, en fin de course du piston, à coopérer avec ladite jupe (13A ; 113A) pour déformer cette dernière de manière à rapprocher une portion de ladite jupe de la partie d'actionnement (19B, 20B ; 119B, 120B) de la patte de retenue (19, 20 ; 119, 120) pour favoriser la coopération entre la jupe et la partie d'actionnement.

2. Dispositif selon la revendication 1, **caractérisé en ce que**, au moins dans la position de retenue de la patte de retenue (19, 20), la partie d'actionnement (19B, 20B) de cette patte de retenue est disposée sensiblement en regard de la rampe de déflexion (30, 32).

3. Dispositif selon la revendication 1 ou 2, **caractérisé en ce que** la partie d'extrémité proximale (16A) du manchon de support (16) forme une cavité cylindrique (36) dans laquelle la jupe cylindrique (13A) du piston (12) est apte à pénétrer en fin de course d'injection de ce dernier et **en ce que** la rampe (30, 32) est formée sur au moins une portion de nervure annulaire disposée sur la paroi axiale interne de ladite cavité.

4. Dispositif selon l'une quelconque des revendications 1 à 3, **caractérisé en ce que** la partie d'actionnement (19B, 20B) de la patte de retenue (19, 20) est formée sur un côté externe de la patte de retenue et **en ce que** la rampe de déflexion (30, 32) est formée sur la périphérie interne du manchon de support (16).

5. Dispositif selon la revendication 4, **caractérisé en ce que** le corps de seringue (10) présente une collerette (10C) située à l'extrémité proximale (10A) de ce corps, **en ce que**, au moins dans sa position de retenue, la patte de retenue (19, 20) s'étend entre cette collerette (10C) et l'extrémité distale (10B) du corps de seringue et **en ce que** la patte de retenue (19, 20) est au moins en partie située dans l'encombrement diamétral de la collerette (10C) lorsque cette patte est sollicitée par la jupe cylindrique (13A) vers sa position libérée.

6. Dispositif selon l'une quelconque des revendications 1 à 5, **caractérisé en ce que** le manchon de protection (18) est emmanché dans le manchon de support (16) et **en ce que** la patte de retenue (19, 20) présente, sur sa périphérie externe, un décrochement de retenue (19A, 20A) permettant sa retenue par rapport à une nervure de retenue (17A) que présente le manchon de support (16).

7. Dispositif selon la revendication 1, **caractérisé en ce que** le manchon de support (116) est emmanché dans le manchon de protection (118), **en ce que** la partie d'extrémité proximale (116A) du manchon de support présente une portion de paroi axiale externe sur laquelle est formée la rampe (130) et **en ce que** la partie d'actionnement (119B) de la patte de retenue (119, 120) est formée sur un côté externe de la patte de retenue.

8. Dispositif selon l'une quelconque des revendications 1 à 7, **caractérisé en ce qu'**il comporte un ressort de rappel (25, 125) disposé entre une première surface d'appui (22' ; 122') fixe par rapport au manchon de support (16 ; 116) et une deuxième surface d'appui (18' ; 118') fixe par rapport au manchon de protection (18, 118), pour solliciter le coulissement relatif de ces manchons.

## Patentansprüche

1. Sicherheitsgerät für eine Spritze, die einen Spritzenkörper (10), einen Kolben (12; 112) und eine Nadel (14) aufweist, wobei das Gerät eine Traghülse (16; 116) hat, die geeignet ist, den Spritzenkörper (10) relativ dazu zu halten, eine Schutzhülse (18; 118) und mindestens eine Haltelasche (19, 20; 119, 120), die mit der Schutzhülse starr verbunden ist und die geeignet ist, eine Halteposition einzunehmen, in der die Haltelasche relativ zur Traghülse gehalten wird, und eine Freigabeposition, in der die Haltelasche relativ zur Traghülse freigegeben wird, um ein relatives Gleiten der Schutzhülse und der Traghülse in Richtung auf eine Schutzposition zu ermöglichen, in der die Nadel (14) von der Schutzhülse (18; 118) umschlossen ist, wobei der Kolben eine zylindrische Schürze (13A; 113A) hat, die am Ende des Injektionshubes des Kolbens gegenüber einem proximalen Endbereich (16A, 116A) der Traghülse (16; 116) zu liegen kommt, für den Eingriff mit einem Betätigungsteil (19B, 20B; 119B, 120B) der Haltelasche, um diese Lasche in Richtung auf ihre Freigabeposition zu beaufschlagen, **dadurch gekennzeichnet, dass** der proximale Endbereich (16A; 116A) der Traghülse (16; 116) mindestens eine Durchfederungsrampe (30, 32; 130) hat, die geeignet ist für den Eingriff mit der zylindrischen Schürze (13A; 113A) am Ende des Kolbenhubes, um die Schürze derart zu verformen, dass ein Teil der Schürze näher an den Betätigungsteil (19B, 20B; 119B, 120B) herangeführt wird, um den Eingriff zwischen der Schürze und dem Betätigungsteil zu fördern.

2. Gerät nach Anspruch 1, **dadurch gekennzeichnet, dass** der Betätigungsteil (19B, 20B) der Haltelasche (19, 20) zumindest in der Halteposition dieser Haltelasche der Durchfederungsrampe (30, 32) im Wesentlichen gegenüberliegend angeordnet ist.

3. Gerät nach Anspruch 1 oder 2, **dadurch gekennzeichnet, dass** der proximale Endbereich (16A) der Traghülse (16) einen zylindrischen Hohlraum (36) bildet, in den sich die zylindrische Schürze (13A) des Kolbens (12) am Ende des Injektionshubes hinein bewegen kann, und dass die Rampe (30, 32) an zumindest einem ringförmigen Rippenbereich gebildet ist, der an der inneren axialen Wand des Hohlraums angeordnet ist.

4. Gerät nach den Ansprüche 1 bis 3, **dadurch gekennzeichnet, dass** der Betätigungsteil (19B, 20B) der Haltelasche (19, 20) an einer Außenseite der Haltelasche gebildet ist und dass die Durchfederungsrampe (30, 32) an der inneren Peripherie der Traghülse (16) gebildet ist.

5. Gerät nach Anspruch 4, **dadurch gekennzeichnet, dass** der Spritzenkörper (10) einen Bund (10C) hat, der sich an dem proximalen Ende (10A) des Körpers befindet und dass sich die Haltelasche (19, 20) zumindest in ihrer Halteposition zwischen dem Bund (10C) und einem distalen Ende (10B) des Spritzenkörpers erstreckt und dass sich die Haltelasche (19, 20) zumindest teilweise in einem radialen Hauptteil des Bundes (10C) befindet, wenn die Lasche durch die zylindrische Schürze (132A) in Richtung auf ihre Freigabeposition beaufschlagt wird.

6. Gerät nach einem der Ansprüche 1 bis 5, **dadurch gekennzeichnet, dass** die Schutzhülse (18) in die Traghülse (16) eingepasst ist und dass die Haltelasche (19) an ihrer äußeren Peripherie einen Haltevorsprung (19A, 20A) hat, durch den sie relativ zu einer Halterippe (17A) der Traghülse (16) gehalten werden kann.

7. Gerät nach Anspruch 1, **dadurch gekennzeichnet, dass** die Traghülse (116) in die Schutzhülse (118) eingepasst ist, dass der proximale Endbereich (116A) der Traghülse einen Bereich einer axial äußeren Wand hat, an dem die Rampe (130) gebildet ist, und dass der Betätigungsteil (119B) der Haltelasche (119, 120) an einer Außenkante der Haltelasche gebildet ist.

8. Gerät nach einem der Ansprüche 1 bis 7, **dadurch gekennzeichnet, dass** dieses eine Rückstellfeder (25, 125) umfasst, die zwischen einer ersten Stützfläche (22'; 122'), die relativ zur Traghülse (16, 116) festgelegt ist, und einer zweiten Stützfläche (18', 118'), die relativ zur Schutzhülse (18, 118) festgelegt ist, angeordnet ist, um diese Hülsen derart zu beaufschlagen, dass sie relativ zueinander gleiten.

## Claims

1. Safety device for a syringe having a syringe body (10), a piston (12; 112) and a needle (14), where the device includes a support sleeve (16; 116) suitable for retaining the body of the syringe (10) relative thereto, a protection sleeve (18; 118) and at least one retaining tab (19, 20; 119, 120) rigidly connected to the protection sleeve and suitable for occupying a retained position in which this tab is retained relative to the support sleeve and a released position in which said tab stops being retained relative to the support sleeve to allow relative sliding of the protection sleeve and the support sleeve towards a protection position in which the needle (14) is surrounded by the protection sleeve (18; 118), where the piston has a cylindrical skirt (13A; 113A) suitable, at the end of the injection stroke of said piston, for coming opposite a proximal end portion (16A; 116A) of the support sleeve (16, 116) for engaging with an actuating part (19B, 20B; 119B, 120B) of the retaining tab for urging said tab towards the released position thereof,
**characterized in that** the proximal end portion (16A; 116A) of the support sleeve (16; 116) has at least one deflection ramp (30, 32; 130) suitable, at the end of the piston stroke, for engaging with said skirt (13A; 113A) for deforming the skirt to bring a portion of said skirt closer to the actuating part (19B, 20B; 119B, 120B) of the retention tab (19, 20; 119, 120) to enhance the engagement between the skirt and the actuating part.

2. Device according to claim 1, **characterized in that** at least in the retained position of the retention tab (19, 20), the actuating part (19B, 20B) of this retention tab is arranged substantially opposite from the deflection ramp (30, 32).

3. Device according to claims 1 or 2, **characterized in that** the proximal end part (16A) of the support sleeve (16) forms a cylindrical cavity (36) into which the cylindrical skirt (13A) of the piston (12) is suited for entering at the end of the injection stroke thereof, and the ramp (30, 32) is formed on at least one annular rib portion arranged on the internal axial wall of said cavity.

4. Device according to claims 1 to 3, **characterized in that** the actuating part (19B, 20B) of the retention tab (19, 20) is formed on an outer side of the retention tab, and the deflection ramp (30, 32) is formed on the inner periphery of the support sleeve (16).

5. Device according to claim 4, **characterized in that** the syringe body (10) has a collar (10C) located at a proximal end (10A) of the body, at least in its retained position, the retention tab (19, 20) extends between the collar (10C) and a distal end (10B) of the syringe body, and the retention tab (19, 20) is at least, in part located in a radial bulk of the collar (10C) when the tab is urged by the cylindrical skirt (13A) towards its released position.

6. Device according to any one of claims 1 to 5, **characterized in that** the protection sleeve (18) is fitted into the support sleeve (16), and the retention tab (19, 20) has, on an outer periphery thereof, a retention projection (19A, 20A) enabling the retention thereof relative to a retention rib (17A) of the support sleeve (16).

7. Device according to claim 1, **characterized in that** the support sleeve (116) is fitted into the protection sleeve (118), the proximal end part (116A) of the support sleeve has a portion of outer axial wall on which the ramp (130) is formed, and the actuation part (119B) of the retention tab (119, 120) is formed on an outer edge of the retention tab.

8. Device according to any one of claims 1 to 7, **characterized in that** it comprises a restoring spring (25, 125) arranged between a first supporting surface (22'; 122') fixed relative to the support sleeve (16, 116) and a second supporting surface (18', 118') fixed relative to the protection sleeve (18, 118) for urging the relative sliding of these sleeves.
